# EUROPEAN PATENT SPECIFICATION

(11) **EP 3 543 250 B1**
(45) Date of publication and mention of the grant of the patent: **07.08.2024**
(21) Application number: 17872682.4
(22) Date of filing: 17.10.2017
(51) Int. Cl.: C07K 1/36, C07K 1/18, C07K 1/34, C12N 9/64

(54) **METHOD FOR REMOVING FXI WHEN PURIFYING PLASMA PROTEINS**
VERFAHREN ZUR ENTFERNUNG VON FXI BEI DER AUFREINIGUNG VON PLASMAPROTEINEN
PROCÉDÉ D'ÉLIMINATION DE FXI LORS DE LA PURIFICATION DE PROTÉINES PLASMATIQUES

(30) Priority: 18.11.2016 KR 20160153716
(43) Date of publication of application: 25.09.2019
(73) Proprietor: GREEN CROSS CORPORATION, Yongin-si, Gyeonggi-do 16924 (KR)
(72) Inventor: KANG, Dae-eun, Yongin-si Gyeonggi-do 16924 (KR); KANG, Gil Bu, Yongin-si Gyeonggi-do 16924 (KR); YOO, Seol Young, Yongin-si Gyeonggi-do 16924 (KR); LEE, Jihye, Yongin-si Gyeonggi-do 16924 (KR); KIM, Ki-Yong, Cheongju-si Chungcheongbuk-do 28119 (KR); KIM, Soo-kwang, Yongin-si Gyeonggi-do 16924 (KR)
(74) Representative: Winter, Brandl - Partnerschaft mbB
(86) International application number: PCT/KR2017/011439
(87) International publication number: WO 2018/093049

(56) References cited:
- WO-A1-00/76534
- JP-A- 2003 511 468
- KR-A- 20160 118 298
- KR-A- 20160 118 298
- KR-A- 20160 118 299
- KR-B1- 101 657 690
- US-A- 5 252 217
- US-A1- 2010 056 766
- US-A1- 2013 058 961
- US-A1- 2013 058 961
- YU-WEN WU ET AL: "Dedicated removal of immunoglobulin (Ig)A, IgM, and Factor (F)XI/activated FXI from human plasma IgG : Removal of IgA, IgM, and FXI from IgG", TRANSFUSION, vol. 54, no. 1, 1 January 2014 (2014-01-01), US, pages 169 - 178, XP055697808, ISSN: 0041-1132, DOI: 10.1111/trf.12243
- PARK DONG HWARN ET AL: "A new manufacturing process to remove thrombogenic factors (II, VII, IX, X, and XI) from intravenous immunoglobulin gamma preparations", BIOLOGICALS, ACADEMIC PRESS LTD., LONDON, GB, vol. 45, 18 November 2016 (2016-11-18), pages 1 - 8, XP029877918, ISSN: 1045-1056, DOI: 10.1016/J.BIOLOGICALS.2016.11.002

## Description

### [Technical Field]

The present invention relates to a method for removing factor XI (FXI) during plasma protein separation and purification, and more specifically to a method for removing FXI including dialyzing and/or concentrating a plasma protein solution containing FXI and various plasma proteins, and then removing the FXI through cation exchange chromatography.

### [Background Art]

A plasma protein is a protein contained in plasma, and representative examples of plasma protein include various blood coagulation factors such as albumin, immunoglobulin and fibrinogen, thrombin, factor 8, alpha 1 antitrypsin and the like. These plasma proteins are separated and purified and then used for various pharmaceutical uses.

FXIa is an activated form of FXI, which activates the downstream factor, FIX, to cause a coagulation cascade in the blood (Wolberg AS et al., AM, J. Hematol., 2000, Vol. 65 (1), pp30-34). Therefore, it is known that plasma protein-derived drugs induce thrombogenesis and thus cause various side effects when they contain these FXIs as impurities during the separation and purification process.

For example, when plasma-derived immunoglobulin (IVIG) is used for pharmaceutical uses, it is necessary to remove FXI so that IVIG does not contain FXI. As can be seen from "Human Normal Immunoglobulin for Intravenous Administration Monograph" of the revised European pharmacopoeia ver. 7.0, removal of FXI is required in order to use plasma proteins for pharmaceutical uses through separation and purification, for example, the process of preparing intravenous immunoglobulin includes a step of removing thrombogenic substances (coagulation factors and zymogens thereof), or data demonstrating that zymogen activation does not occur during the process is needed (Human Normal Immunoglobulin for Intravenous Administration, European Pharmacopeia 7.0, PA/PH/Exp. 6B/T(11) 14PUB, 2012 Jan. Report No. 01/2012:0918) .

KR 101 657 690 B1 discloses methods for preparing hepatitis B immunoglobulin derived from plasma.

KR 2016 0118298 A describes methods for purifying immunoglobulin.

US 2013/0058961 A1 provides methods for reducing the amidolytic and anti-complement activity (ACA) content of an immunoglobulin composition through the use of cation exchange chromatography.

However, it is difficult to efficiently and completely remove FXI through methods known to date.

Accordingly, as a result of efforts to solve the above-mentioned problems, the present inventors have found that when a fraction II paste isolated from plasma containing FXI and various plasma proteins is dialyzed and concentrated, and is then purified through cation exchange chromatography, thrombogenic substances such as FXI present in the plasma can be efficiently removed. Based on this finding, the present invention has been completed.

### [Disclosure]

### [Technical Problem]

Therefore, it is an object of the present invention to provide a method for removing factor XI (FXI) during plasma protein purification.

### [Technical Solution]

In accordance with the present invention, the above and other objects can be accomplished by the provision of a method for removing factor XI (FXI) during plasma protein separation and purification, including (a) obtaining a plasma protein solution containing FXI and a plasma protein, (b) dialyzing and/or concentrating the obtained solution, (c) treating the concentrated solution with a solvent and a detergent, followed by performing cation exchange chromatography to bind the FXI and the plasma protein to a column, and (d) selectively eluting only the plasma protein.

Moreover, the cation exchange chromatography of step (c) is carried out at a pH of 4.5 to 5.5 and at a flow rate of 60 cm/hr, and steps (c) and (d) are carried out at a temperature of 18 to 25°C.

### [Brief Description of the Drawings]

The above and other objects, features and other advantages of the present invention will be more clearly understood from the following detailed description taken in conjunction with the accompanying drawings, in which:

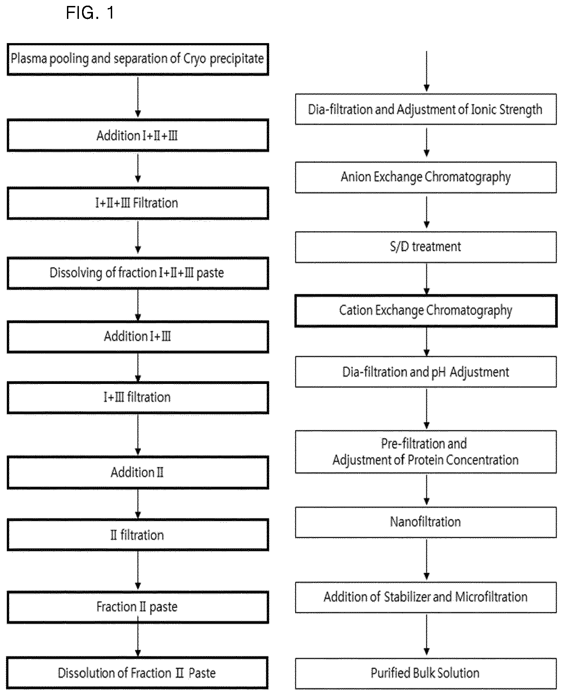

FIG. 1 is a schematic diagram illustrating a process for preparing an immunoglobulin for intravenous injection according to the present invention; and
FIG. 2 shows the results of measurement of the concentration of FXI (human coagulation factor XI) contained in a filtrate or precipitate by SDS-PAGE and Western blotting in each preparation step.

### [Detailed Description of the Invention]

Unless defined otherwise, all technical and scientific terms used herein have the same meanings as appreciated by those skilled in the field to which the present invention pertains. In general, the nomenclature used herein is well-known in the art and is ordinarily used.

As used herein, the term "plasma protein" includes cryo-poor plasma containing various plasma proteins such as FXI (factor XI, factor 11) and thrombin in human plasma or human placenta-derived plasma, various Cohn fractions, and ammonium sulfate or fractions obtained through precipitation by PEG (Polson et al., Biochem. Biophys. Acta, 82:463, 1964; Polson and Ruiz-Bravo, Vox Sang, 23:107. 1972). Preferably, the plasma protein fraction may also be Cohn fraction II; Cohn fraction I, II and III; or Cohn fraction II + III.

In one embodiment of the present invention, a fraction II paste obtained from human plasma was used and prepared according to a conventional Cohn plasma fractionation method. Subsequent purification steps were conducted to remove various lipoproteins, fibrinogen, α-globulin, β-globulin and various coagulation factors (such as factor XI) contained in the fraction II paste.

Accordingly, in one aspect, the present invention is directed to a method for removing factor XI (FXI) during plasma protein separation and purification including (a) obtaining a plasma protein solution containing FXI and a plasma protein, (b) dialyzing and/or concentrating the obtained solution, (c) treating the concentrated solution with a solvent and a detergent, followed by performing cation exchange chromatography to bind the FXI and the plasma protein to a column, and (d) selectively eluting only the plasma protein.

Moreover, the cation exchange chromatography of step (c) is carried out at a pH of 4.5 to 5.5 and at a flow rate of 60 cm/hr, and steps (c) and (d) are carried out at a temperature of 18 to 25°C.

The human plasma herein used is plasma obtained from the Korean Red Cross approved by FDA through biotests including nucleic acid amplification tests (NATs) and serological tests against human immunodeficiency virus (HIV), hepatitis C virus (HCV), hepatitis B virus (HBV) and parvovirus B19. The plasma stored at -20°C or below was dissolved by reaction in a jacketed vessel at 1 to 6°C for 12 to 72 hours.

Under these conditions, as plasma is dissolved, a cryoprecipitate containing fibrinogen, coagulation factors and FXI is produced. Frozen and precipitated plasma is removed through centrifugation and cryo-poor plasma free of the frozen and precipitated plasma is recovered. Then, precipitation and filtration are repeated to finally obtain a fraction II paste.

The filtration for separating plasma containing FXI and plasma proteins is performed by mixing a filtering aid with the plasma and separating a supernatant and a precipitate using a filter press. The filtering aid used herein was Celite (STD), Harbolite (expanded perlite product) or the like.

In the present invention, the plasma protein solution in step (a) may be used without limitation as long as it is a solution containing a plasma protein. Preferably, the plasma protein solution is a fraction II solution obtained by dissolving a plasma protein fraction II paste and then filtering.

In the present invention, the dissolution of the plasma protein fraction II paste may be performed by adding a sodium chloride solution in an amount corresponding to 2 to 10 times the plasma protein fraction volume.

The plasma protein fraction is preferably suspended (dissolved) in water and/or a buffer at a substantially non-denaturing temperature and pH. "Substantially non-denaturing" means that the above-mentioned conditions do not cause substantially irreversible loss of functional activity of plasma proteins such as immunoglobulin molecules, for example, loss of antigen binding activity and/or biological Fc-action.

The plasma protein to be separated and purified in the present invention is preferably an immunoglobulin, but the present invention is not limited thereto.

As an embodiment of the present invention, a method for removing FXI during separation and purification of immunoglobulin will be described in detail.

The plasma protein fraction is dissolved in water acidified with at least one non-denaturing buffer at a volume of 2 to 5 times, preferably 3 to 4 times the plasma protein fraction volume, and the pH of the immunoglobulin-containing suspension is preferably maintained at 6.0 or less, more preferably 4.0 to 6.0, and most preferably 4.1 to 4.3 to secure an optimal solubility of immunoglobulin. Any acidic buffer known in the art may be used as the acidic buffer, but sodium phosphate, sodium acetate, sodium chloride, acetic acid, hydrochloric acid, water (distilled water) or the like may be used. In the present invention, a sodium chloride solution is used.

The immunoglobulin suspension is maintained at a low temperature so as to prevent protein denaturation and minimize protease activity, and the immunoglobulin suspension and water or buffer added are preferably maintained at 0 to 12°C, more preferably at 0 to 7°C, and most preferably at 1 to 4°C.

In the present invention, the filtration is a step for obtaining a fraction II solution, is clarifying filtration, and is performed in the state in which the pH is adjusted to 4.5 to 5.5, preferably 4.9 to 5.1.

In the present invention, the fraction II paste is transferred to a jacketed vessel having a temperature of 10°C or less and then dissolved in a 0.6% sodium chloride solution in an amount of 4 times of the fraction II paste volume, and 1M acetic acid is added to the resulting solution to adjust the pH to 5.0 ± 1. The solution is then clarifying-filtered using a deep filtration cartridge to obtain a fraction II solution.

In the present invention, the dialysis and/or concentration of step (b) may be performed using an ultrafiltration/diafiltration (UF/DF) system and is conducted until the osmotic pressure of the dialysis concentrate is adjusted to 10 mOsmol/kg and the pH is then adjusted to 5.5 to 6.5, preferably to 5.9 to 6.1.

Diafiltration is a combination of dialysis and ultrafiltration, which refers to a method of removing only some solute from a fluid containing two or more solutes with different molecular sizes, is effective in purifying polymers, and has advantages of reducing time and costs compared to conventional dialysis methods.

In one embodiment of the present invention, the fraction II solution was filtered at 10 mOsmol/kg or less using an ultrafiltration/diafiltration (UF/DF) system. 1M sodium acetate was added to the filtrate to give 5.0 ± 1.0 mM and the pH was adjusted to 6.0 ± 0.1 to obtain a dialyzed and/or concentrated aqueous solution containing immunoglobulin.

In the present invention, step (c) aims at inactivating a virus such as a potential lipid enveloped virus in a solution containing an immunoglobulin, and after inactivation, removing the substances used for inactivation and remaining FXI, and may be conducted by treatment with a virus-inactivating agent, preferably a solvent and/or a detergent, most preferably a solvent-detergent mixture.

Potential lipid envelope viruses such as HIV1 and HIV2, hepatitis type C and non A-B-C, HTLV1 and HTLV2, herpes virus group, CMV and Epstein-Barr virus are inactivated through step (c) and thus the safety of the final product can be improved.

Any solvent and detergent can be used in step (c) without limitation as long as they are capable of inactivating a virus, particularly a lipid envelope virus. The detergent may be selected from the group consisting of non-ionic and ionic detergents, and is preferably substantially non-denaturing. Particularly, in consideration of ease of removal, nonionic detergents are preferred, and the solvent is most preferably tri-n-butyl phosphate (TNBP), as disclosed in U.S. Patent No. 4,764,369, but the present invention is not limited thereto.

A particularly preferred virus-inactivating agent for implementing the invention is a mixture of TNBP and one or more selected from polysorbate 80 (Tween 80), Triton X-100 and Triton X-45, but the present invention is not limited thereto.

A preferred solvent/detergent mixture is added such that the concentration of TNBP in the immunoglobulin-containing solution is within the range of 0.2 to 0.6% by weight, preferably the range of 0.24 to 0.36% by weight, and the concentration of Tween 80 is within the range of 0.8 to 1.5% by weight, preferably the range of 0.8 to 1.2% by weight.

The virus-inactivation step is carried out under the condition that the lipoic envelope virus is deactivated to produce an immunoglobulin-containing solution that has substantially no risk of containing a virus. The reaction temperature is preferably 4 to 30°C, more preferably 19 to 28°C, and most preferably 24 to 26°C under the above conditions. The reaction time is preferably 1 to 24 hours, more preferably 4 to 12 hours, and most preferably about 8 hours, which is sufficient to ensure virus inactivation.

In the present invention, the cation exchange chromatography of step (c) is carried out at a pH of 4.5 to 5.5 and a flow rate of 60 cm/hr, and preferably at a pH of 4.9 to 5.1. The immunoglobulin loaded on the cation exchange resin is from 90 to 130 mg per mL of the cation exchange resin, more preferably from 95 to 105 mg per mL of the cation exchange resin, and most preferably 84 mg per mL of the cation exchange resin. The immunoglobulin is absorbed and washed with an equilibration buffer. The equilibration buffer used for washing under the above condition can be used in a volume of 3 times or more, preferably 5 times or more of the column volume. After washing, immunoglobulin is eluted with an elution buffer of 8 times or more of the column volume.

The cation exchange resin may be Sephardex, Sepharose, HyperCell, Source, or the like, but is not limited thereto. Other cation exchange resins known in the art can be used. Particularly, in the present invention, a ceramic-based cation exchange resin is preferably used. In one embodiment of the present invention, a ceramic-based CM hyper D gel is used as a cation exchange resin. The column buffer herein used is an equilibration buffer, wash buffer or elution buffer well-known in the art, such as a sodium phosphate buffer, a citric acid buffer or an acetic acid buffer.

The elution of the immunoglobulin from the cation exchange resin according to step (d) is performed using a substantially non-denaturing buffer having a pH and ionic strength sufficient to cause efficient elution of immunoglobulin, thereby recovering an immunoglobulin-containing eluate. The term "efficient elution" means that 75% or more, 80% or more, 85% or more or the like of the immunoglobulin solution loaded in the cation exchange resin is eluted from the cation exchange resin.

In the present invention, the elution of the immunoglobulin in step (d) may be carried out at a salt concentration of the elution buffer that is high enough to replace the immunoglobulin in the cation exchange resin, and the elution of the immunoglobulin in step (d) may be carried out at a salt concentration of 400 to 600 mM, preferably 500 mM. The salt is preferably sodium chloride (NaCl), but the present invention is not limited thereto.

In the present invention, the method may further include subjecting the cation exchange resin to cleaning in place (CIP) using sodium hydroxide having a salt concentration of 200 to 1,000 mM, preferably 500 mM, after the completion of the elution of plasma protein in step (d).

In the present invention, the cation exchange chromatography in step (c) and the elution in step (d) are carried out at a temperature of 18 to 25°C, more preferably 19 to 23°C, and most preferably 19.5 to 22.5°C.

In the present invention, the method may further include conducting anion exchange chromatography to obtain a fraction not attached to the anion exchange chromatography column, between steps (b) and (c).

In the present invention, the anion exchange chromatography is conducted under conditions of a pH of 5.5 to 6.5 and a flow rate of 95 to 145 cm/hr, and a fraction not attached to the anion exchange chromatography column is obtained in a loading volume (LV) of 1.5 to 2.0.

The anion exchange resin used in the anion exchange chromatography step may be an anion exchange resin substituted with diethylaminoethyl (DEAE) or quaternary ammonium, but is not limited thereto. Preferably, any one may be selected from an anion exchange resin having strongly basic quaternary ammonium and an anion exchange resin having weakly basic diethylaminoethyl (DEAE).

Examples of strongly basic anion exchange resins include, but are not limited thereto, Q Sepharose Fast Flow, Q Sepharose High Performance, Resource Q, Source 15Q, Source 30Q, Mono Q, Mini Q, Capto Q, Capto Q ImpRes, Q HyperCel, Q Ceramic HyperD F, Nuvia Q, UNOsphere Q, Macro-Prep High Q, Macro-Prep 25 Q, Fractogel EMD TMAE(S), Fractogel EMD TMAE Hicap (M), Fractogel EMD TMAE (M), Eshmono Q, Toyopearl QAE-550C, Toyopearl SuperQ-650C, Toyopearl GigaCap Q-650M, Toyopearl Q-600C AR, Toyopearl SuperQ-650M, Toyopearl SuperQ-650S, TSKgel SuperQ-5PW (30), TSKgel SuperQ-5PW (20), TSKgel SuperQ-5PW and the like. Any anion exchange resin known in the art can be used.

The appropriate volume of the resin used for anion exchange chromatography is determined in consideration of the column dimensions, *i.e.,* the diameter of the column and the height of the resin, and depends on, for example, the amount of immunoglobulin solution in the applied solution and the binding performance of the resin used. Prior to conducting ion exchange chromatography, the ion exchange resin is preferably equilibrated with a buffer to allow the resin to bind to a counter ion thereof.

In the present invention, a DEAE Sepharose gel is used as the anion exchange resin. The column buffer used herein may be an equilibration buffer, a wash buffer, or an elution buffer well-known in the art such as a sodium phosphate buffer, a citric acid buffer or an acetic acid buffer.

In anion exchange chromatography, the column is equilibrated to a pH of 6.0 ± 0.1 with 5 ± 1.0 mM sodium acetate buffer, and the flow rate of the mobile phase is adjusted to 95 to 145 cm/hr. A fraction not attached to the anion exchange chromatography column is recovered in a loading volume (LV) of 1.5 to 2.0.

Hereinafter, the present invention will be described in more detail with reference to examples. However, it will be obvious to those skilled in the art that these examples are suggested only for illustration of the present invention and should not be construed as limiting the scope of the present invention.

### <Example 1> Removal of factor XI upon preparation of immunoglobulin for intravenous injection.

### 1-1: Plasma preparation

The plasma used herein was plasma approved by the FDA through biotests including nucleic acid amplification tests (NATs) and serological tests on human immunodeficiency virus (HIV), hepatitis C virus (HCV), hepatitis B virus (HBV) and parvovirus B19.

In the present invention, plasma obtained from the Red Cross (Batch No. 600A9008) was used and stored at -20°C or lower before use. The bottle containing plasma was opened with a bottle-cutting machine and the plasma was dissolved through reaction in a jacketed vessel at 1 to 6°C for 12 to 72 hours.

As the plasma was dissolved under these conditions, a cryoprecipitate containing fibrinogen and coagulation factors was produced. The frozen and precipitated plasma was removed through centrifugation (under the conditions described above) and cryo-poor plasma free of the frozen and precipitated plasma was recovered.

### 1-2: Precipitation I step

A precipitation I step was conducted in order to further remove the coagulation factor from the frozen deficient plasma.

96% ethanol was added to the frozen deficient plasma recovered in Example 1-1 such that the final ethanol concentration became 8 ± 0.8% at -3 ± 1°C, and then the pH was adjusted to 7.2 ± 0.2 using an acetate buffer. The precipitate was removed through centrifugation (under conditions described above), an addition I supernatant was recovered, and a portion thereof was sampled and stored to obtain a total viable count.

### 1-3: Precipitation II + III step and filtration

The precipitation II + III step was conducted in order to precipitate immunoglobulin contained in the supernatant recovered in Example 1-2.

96% ethanol was added to the supernatant recovered in Example 1-2 such that the final ethanol concentration became 20 ± 2% at -5 ± 1.0°C, and the pH was adjusted to 6.9 ± 0.1 using an acetate buffer.

Then, 0.0284 kg of a filtering aid (Celite (STD)) and 0.0284 kg of Harbolite (expanded perlite product)) per kg of plasma were added and mixed for 30 ± 10 minutes. The supernatant and the precipitate were separated from the mixture using a filter press (DG800K) in a cold room maintained at 2 to 8°C.

The supernatant was designated as "supernatant I + II + III (or II + III)" and the precipitate was designated as "fraction I + II + IIIw (or II + IIIw)" (w; wash). The fraction I + II + IIIw (or II + IIIw) was used immediately or stored at -20°C or lower.

### 1-4: Precipitation III step and filtration

A precipitation III step was conducted in order to further remove albumin, lipoprotein, thrombin and other unwanted proteins from the fraction I + II + IIIw (or II + IIIw) containing immunoglobulin.

The fraction I + II + IIIw (or II + IIIw) recovered in Example 1-3 was dissolved in cold distilled water and a portion thereof was sampled and stored to obtain a total viable count. 96% ethanol was added to the fraction I + II + IIIw (or II + IIIw) dissolved in distilled water such that the final ethanol concentration became 18 ± 1.8% at -5 ± 1.0°C, and the pH was adjusted to 5.2 ± 0.1 with a -6°C acetate buffer that had been previously prepared.

The supernatant and the precipitate were separated from the mixture using a filter press (DG800K). The supernatant was designated as "filtrate I + III (or III)" and the precipitate was designated as "fraction I + III (or III)". The fraction I + III (or III) was discarded and a portion of the filtrate I + III (or III) was sampled and stored in order to obtain a total viable count.

### 1-5: Precipitation II step and filtration

The precipitation II step was conducted in order to precipitate the immunoglobulin from the filtrate I + III (or III) of Example 1-4.

96% ethanol was added to the filtrate I + III (or III) such that the final ethanol concentration became 25 ± 2.5% at -10 ± 2.0°C and the pH was adjusted to 7.4 ± 0.2 using 1M sodium bicarbonate.

The supernatant and the precipitate were separated from the mixture using a filter press (DG800K). The precipitate was designated as "fraction II paste" and a portion of the fraction II paste was sampled and stored for later determination of contents and compositions of bacterial endotoxins and proteins.

### 1-6: Fraction II paste dissolution and clarifying filtration

A separation-purification step was conducted in order to increase the content of immunoglobulin isolated from plasma and to remove thrombogenic substances.

First, the fraction II paste containing immunoglobulin isolated in Example 1-5 was dissolved in order to satisfy the conditions suitable for dialysis. The fraction II paste was transferred to a jacketed vessel at 10°C or less and was dissolved in a 0.6% sodium chloride solution of 4 times the volume of the fraction II paste. A portion of the resulting fraction II paste solution was sampled and stored to determine the content and composition of proteins.

Then, the pH of the dissolved solution was adjusted to 5.0 ± 1 using 1M acetic acid, and the solution was subjected to clarifying filtration using a depth filter cartridge (BecodiskBP01) to obtain a fraction II solution.

### 1-7: Diafiltration

Ethanol and low molecular ions were removed from the fraction II solution containing immunoglobulin obtained in Example 1-6, and the pH was adjusted in order to satisfy conditions suitable for anion exchange chromatography.

The fraction II solution containing immunoglobulin was subjected to diafiltration at 10 mOsmol/kg or less using an ultrafiltration/diafiltration [Millipore Pellicon2 (50K)] system. A portion of the filtrate thus obtained was sampled and stored for later determination of protein content, composition and viable cell count.

1M sodium acetate was added to the filtrate to give 5.0 ± 1.0 mM and the pH was adjusted to 6.0 ± 0.1 to obtain a dialyzed and/or concentrated immunoglobulin solution.

### 1-8: Anion exchange chromatography

Anion exchange chromatography was conducted in order to remove polymers and other plasma proteins contained in the dialyzed and/or concentrated immunoglobulin solution obtained in Example 1-7.

The anion exchange resin was charged in a DEAE Sepharose gel column (GE Healthcare, Catalog No. 17-0709) and equilibrated to a pH of 6.0 ± 0.1 using an equilibration buffer. Then, the dialyzed and/or concentrated immunoglobulin solution obtained in Example 1-7 was loaded on the column at a flow rate of 120 ± 25 cm/hr and the fraction not attached to the anion exchange chromatography column was recovered in a loading volume (LV) of 1.5 to 2.0.

### 1-9: Solvent/detergent treatment for virus inactivation

The step of treating with a solvent and a detergent was conducted in order to inactivate the potential lipid enveloped virus of the solution containing immunoglobulin.

First, acetic acid was added to the fraction not attached to the anion exchange chromatography column recovered in Example 1-8 to adjust the pH to 5.0 ± 0.1, tri(n-butyl)-phosphate (TNBP) and Polysorbate 80 (Tween 80) were added to give concentrations of 0.3 ± 0.06% and 1 ± 0.2%, respectively, and the mixture was stirred at 200 ± 50 rpm for 20 to 30 minutes. A portion of the solution was sampled to identify whether or not TNBP and Tween 80 were homogeneously mixed in the solution, and then the mixture was continuously stirred at 200 ± 50 RPM at 25 ± 1.0°C for 8 hours. After the stirring was completed, the solution containing immunoglobulin was transferred through a hard pipeline to another tank, which was a viral secure area (VSA) .

### 1-10: Cation exchange chromatography

Cation exchange chromatography was conducted at 21 ± 1.5°C in order to remove TNBP, Tween 80 and other thrombogenic substances such as coagulation factors from the solvent/detergent-treated immunoglobulin solution.

The cation exchange resin was charged in a column of CM hyper D gel (Pall Corporation, Catalog No. 20050), which was made of a ceramic material, and equilibrated to a pH of 5.0 ± 0.1 using an equilibration buffer. Then, the solvent/detergent-treated immunoglobulin solution of Examples 1-9 was loaded onto the column at a flow rate of 60 cm/hr. Then, the column was washed with a wash buffer of 7 times the volume of the column and was recovered by eluting (adsorption rate: 84 mg of immunoglobulin per 1 ml of cation exchange resin) the immunoglobulin using an elution buffer (elution buffer composition: 20 mM NaOAc pH 4.5 w/0.5 M NaCl).

### <Example 2> Removal of FXI through CIP after cation exchange chromatography

The column used in Example 1-10 was flowed with 2M NaCl (CIP1) in a volume equal to 2 times of the column volume and then with 0.5M NaOH (CIP2) in a volume equal to 2 times of the column volume. Then, CIP1 and CIP2 were collected and FXI (FXIa) contents was measured in order to identify the removal of FXI (FXIa) during cation exchange chromatography.

As a result, FXI (FXIa) was measured at 2.9% of the load sample from 2M NaCl in the first CIP step and FXI (FXIa) was measured in an amount less than a detection limit (0.31 ng/mL) from 0.5M NaOH in the second CIP step (Table 1).

The use of NaOH for CIP of a CM Ceramic HyperD F resin is recommended and NaOH is generally widely used for a resin CIP process. Therefore, it was assumed that FXI (FXIa) would be removed from 0.5M NaOH in the second CIP step, but FXI (FXIa) was not measured in the ELISA test due to the harsh conditions. In fact, it has been reported that NaOH causes protein degradation through peptide bond hydrolysis.

**[Table 1]**

| Identification of FXI removal: General CIP process | | | | |
|---|---|---|---|---|
| **FXI(FXIa) content** | **Conc. (ng/mL)** | **Volume (mL)** | **Total (ng)** | **Residual ratio(%)** |
| Load(656B14012) | 421.45 | 225 | 94825.1 | 100 |
| Flow through | <0.31 | 650 | <201.5 | - |
| Elution | <0.31 | 450 | <139.5 | - |
| 2M NaCl (CIP 1) | 26.30 | 104 | 2735.3 | 2.9 |
| 0.5M NaOH (CIP 2) | <0.31 | 82 | <25.4 | - |

In order to identify that FXI (FXIa) was bound during the process (flow through, elution) and was eluted during the CIP, based on the results shown in Table 1, CIP was conducted using 6M guanidine HCl, instead of 0.5M NaOH. 6M guanidine HCl is mainly used as a well-known protein denaturant for protein purification. As a result of CIP using 6M guanidine HCl, it was found that FXI (FXIa) was eluted at 59.3%.

Thus, it was found that FXI (FXIa) was not eluted but was bound during the CM chromatography process (Table 2).

**[Table 2]**

| Identification of FXI removal: Modified CIP process | | | | |
|---|---|---|---|---|
| **FXI(FXIa) content** | **Conc. (ng/mL)** | **Volume (mL)** | **Total (ng)** | **Residual ratio (%)** |
| Load (656B14012) | 410.16 | 225 | 92285.1 | 100 |
| Flow through | <0.31 | 645 | <200.0 | - |
| Elution | <0.31 | 450 | <139.5 | - |
| 2M NaCl | 11.33 | 106 | 1200.5 | 1.3 |
| 6M guanidine HCl | 684.40 | 80 | 54752.2 | 59.3 |

The content of FXI (FXIa) in the sample was measured using the FXI content test. As a result, the content of FXI (FXIa) in the load sample was 418.7 ng/mL and the content of FXI in the elution sample was less than a detection limit (0.31 ng/mL) after cation exchange chromatography. After conducting a CIP process using 0.5 NaOH, the CIP process was further conducted using 6M guanidine HCl in order to determine whether or not the FXI (FXIa) was completely removed.

As a result, FXI (FXIa) was measured in an amount less than the detection limit (0.31 ng/mL) in 6M guanidine HCl, which indicates that FXI (FXIa) was completely removed using 0.5M NaOH.

For each step, the concentration of FXI was measured by ELISA.

**[Table 3]**

| Identification of FXI removal: Identification process | | | | |
|---|---|---|---|---|
| **FXI(FXIa) content** | **Conc. (ng/mL)** | **Volume (mL)** | **Total (ng)** | **Residual ratio (%)** |
| Load (656B14012) | 418.70 | 225 | 94207.5 | 100 |
| Flow through | <0.31 | 645 | <200.0 | - |
| Elution | <0.31 | 435 | <134.9 | - |
| 2M NaCl | 9.77 | 104 | 1016.4 | 1.1 |
| 0.5M NaOH | <0.31 | 102 | <31.6 | - |
| 6M guanidine HCl | <0.31 | 85 | <26.4 | - |

### <Example 3> Measurement of concentration of FXI (human coagulation factor XI) contained in filtrate or precipitate during each preparation step

In order to determine the degree of removal of the thrombus coagulant, the concentration of FXI (Human Coagulation Factor XI) contained in the filtrate or the precipitate in each preparation step of Example 1 was measured by ELISA (Human Factor XI ELISA kit: Innovative, Cat No. IHFXIKT-TOT), SDS-PAGE and western blotting, and the content of FXIa was measured by a TGA test (CBER ver.3).

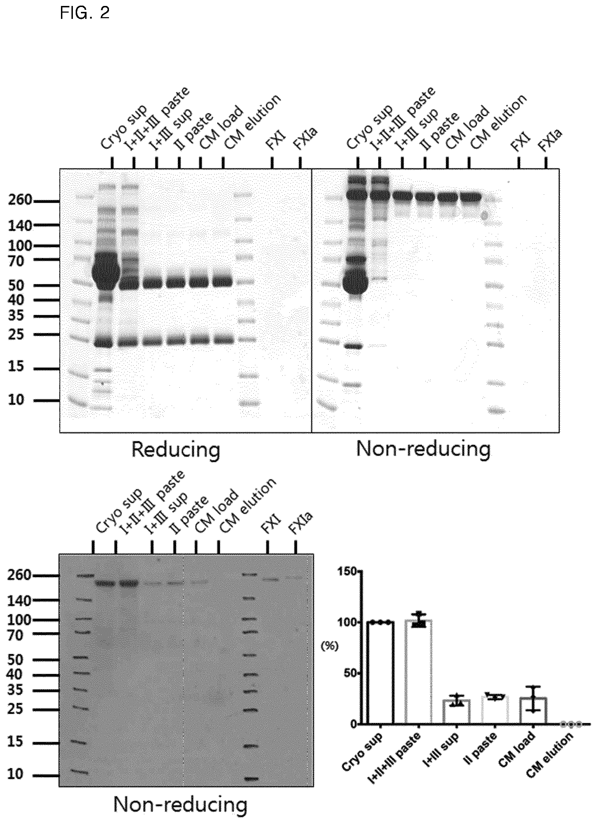

Results of measurement of the FXI contents of the products of the purification process of the present invention showed that there was no change in the anion exchange resin chromatography, as shown in FIG. 2, but FXI was not detected below a detection limit from the elution solution during the cation exchange resin chromatography process, and that all the FXI was removed during the corresponding process.

### <Example 4> Measurement of removal efficiency of human coagulation factor XI (FXI)

In general, the concentration of FXI present in the plasma is known to be 4 to 6 ug/mL. Based on this, the CM load sample was prepared such that the concentration of FXIa was adjusted to 4 ug/mL and a spiking test was then performed. This concentration corresponds to 10 times the concentration of approximately 400 ng/mL measured in the CM load sample (656B14012).

The concentration of FXI (FXIa) in the load sample was measured using the FXI content test. As a result, it was found that the content of FXI (FXIa) in the load sample was 4020.1 ng/mL and FXI (FXIa) was measured in an amount of less than the detection limit (0.31 ng/mL) in the elution sample after cation exchange chromatography. The FXIa activity in the sample was measured by TGA. As a result, the content of FXIa in the load sample was 72837.4 mU/mL and FXIa was measured in an amount of less than a detection limit (0.156 mU/mL) in the elution sample after the cation exchange chromatograph process. This indicates that excessive FXI can be removed through cation exchange chromatography (Table 4).

**[Table 4]**

| Measurement of FXI removal efficiency | | | | |
|---|---|---|---|---|
| **FXI(FXIa) content** | **Conc. (ng/mL)** | **Volume (mL)** | **Total (ng)** | **Residual ratio (%)** |
| Load (656B14012) | 4020.12 | 225 | 904527.5 | 100 |
| Flow through | <0.31 | 645 | <200.0 | - |
| Elution | <0.31 | 435 | <134.9 | - |
| 2M NaCl | 132.18 | 106 | 14011.5 | 1.5 |
| 0.5M NaOH | <0.31 | 80 | <24.8 | - |

In addition, the total amount of FXI present in the cryo-poor plasma (Cryo SUP), which is the starting material of the fractionation process, is about 8 g. A total of 16 g of FXI remains in the process product (8 g x 2 batch = 16 g), assuming that the FXI is not removed during a fractionation process, but is subjected to a purification process in its entirety. When 16 g is divided by 3162 as a scale-down factor of the cation exchange chromatography process, 5 mg is obtained. Thus, the total amount of FXIa in the CM load sample was adjusted to 5 mg and the spiking test was performed (16 g ÷ 3162 ≒ 0.005 g = 5 mg)

This corresponds to about 53 times of 95000 ng which is the total amount measured in an actual CM load sample (656B14012). As a result of measuring the content of FXI (FXIa) in the process sample using the FXI content test, the total FXI (FXIa) in the load sample was about 4.7 mg, and the total FXI (FXIa) in the elution sample after the cation exchange chromatograph process was about 1.3 ug. The residual ratio was 0.0%, which means that more than 99.9% of the load sample was removed (Table 5).

**[Table 5]**

| Measurement of FXI removal efficiency | | | | |
|---|---|---|---|---|
| **FXI(FXIa) content** | **Conc. (ng/mL)** | **Volume (mL)** | **Total (ng)** | **Residual ratio (%)** |
| Load (654B14408) | 21002.15 | 225 | 4725484.2¹⁾ | 100 |
| Flow through | <0.31 | 650 | <201.5 | - |
| Elution | 2.93 | 445 | 1302.5 | 0.0 |
| 2M NaCl | 1155.19 | 106 | 122450.2 | 2.6 |
| 0.5M NaOH | <0.31 | 80 | <24.8 | - |

### <Example 5> Measurement of FXI removal efficiency in sample containing excessive FXI

The removal efficiency of FXI was measured when the cryo poor plasma sample (Cryo sup) and the fraction I + II + III paste sample of the fraction stage, which had not been subjected to a purification step, were subjected to CM hyper D chromatography in the same manner as in Example 3. These samples of two steps contained an excess of FXI.

As a result, as can be seen from Table 6 and Table 7, about 99% of FXI was removed.

**[Table 6]**

| Measurement of removal efficiency in sample containing excess FXI - Cryo free plasma (Cryo sup) loading | | | | |
|---|---|---|---|---|
| **FXI(FXIa) content** | **Conc. (ng/mL)** | **Vol. (mL)** | **Total (ng)** | **Residual ratio (%)** |
| Load (Cryo sup) | 2602.9 | 225 | 585652.5 | 100 |
| FT | 4.5 | 225 | 1012.5 | 0.2 |
| Wash | 0.7 | 395 | 276.5 | 0.0 |
| Elution | 36.3 | 450 | 16335.0 | 2.8 |

**[Table 7]**

| Measurement of removal efficiency in sample containing excess FXI - I + II + III paste loading | | | | |
|---|---|---|---|---|
| **FXI(FXIa) content** | **Conc. (ng/mL)** | **Vol. (mL)** | **Total (ng)** | **Residual ratio (%)** |
| Load (I+II+III paste) | 1779.2 | 225 | 400320.0 | 100 |
| FT | 1.1 | 225 | 247.5 | 0.1 |
| Wash | 1.3 | 395 | 513.5 | 0.1 |
| Elution | 6.8 | 450 | 3060.0 | 0.8 |

Although specific configurations of the present invention have been described in detail, those skilled in the art will appreciate that preferred embodiments of this description are given for illustrative purposes and should not be construed as limiting the scope of the present invention. Therefore, the substantial scope of the present invention is defined by the accompanying claims and equivalents thereto.

### [Industrial availability]

The FXI removal method according to the present invention is capable of increasing the removal efficiency of impurities and thrombogenic substances and maintaining polymer contents, thereby producing stable plasma proteins, particularly immunoglobulin, with improved quality.

## Claims

1. A method for removing factor XI (FXI) during plasma protein separation and purification, comprising:
(a) obtaining a plasma protein solution containing FXI and a plasma protein;
(b) dialyzing and/or concentrating the obtained solution;
(c) treating the concentrated solution with a solvent and a detergent, followed by performing cation exchange chromatography to bind the FXI and the plasma protein to a column; and
(d) selectively eluting only the plasma protein,
wherein the cation exchange chromatography of step (c) is carried out at a pH of 4.5 to 5.5 and at a flow rate of 60 cm/hr, and
wherein steps (c) and (d) are carried out at a temperature of 18 to 25°C.

2. The method according to claim 1, further comprising conducting anion exchange chromatography to obtain a fraction not adhered to an anion exchange chromatography column between step (b) and step (c).

3. The method according to claim 1, wherein the solution of step (a) is obtained by dissolving a plasma protein fraction II paste, followed by filtration.

4. The method according to claim 3, wherein the dissolving the fraction II paste is carried out by adding a sodium chloride solution in a volume equal to 2 to 10 times of the plasma protein fraction volume.

5. The method according to claim 3, wherein the filtration is carried out by clarifying filtration at a pH adjusted to 4.5 to 5.5.

6. The method according to claim 1, wherein the dialysis and/or concentration of step (b) is carried out using an ultrafiltration/diafiltration (UF/DF) system, and is conducted at an osmotic pressure of 10 mOsmol/kg or less and a pH is then adjusted to 5.5 to 6.5.

7. The method according to claim 1, wherein the solvent of step (c) is tri(n-butyl)-phosphate (TNBP) and a detergent comprises one or more selected from polysorbate 80, Triton X-100 and Triton X-45.

8. The method according to claim 1, wherein the cation exchange chromatography of step (c) is carried out at a salt concentration of 400 to 600 mM.

9. The method according to claim 1, wherein an amount of protein adsorbed on the column of the cation exchange chromatography of step (c) is 90 to 130 mg/mL per mL of a cation exchange resin.

10. The method according to claim 1, wherein the cation exchange chromatography of step (c) is carried out using a ceramic-based cation exchange resin.

11. The method according to claim 1, wherein, in step (d), only the plasma protein is selectively eluted using 400 to 600 mM sodium chloride (NaCl).

12. The method according to claim 1, further comprising conducting cleaning in place (CIP) to wash the cation exchange resin using sodium hydroxide (NaOH) after completion of elution of the plasma protein in step (d).

13. The method according to claim 12, wherein the cleaning in place (CIP) is carried out at a sodium chloride concentration of 200 to 1,000 mM.

14. The method according to claim 2, wherein the anion exchange chromatography is carried out at a pH of 5.5 to 6.5 and at a flow rate of 95 to 145 cm/hr and a fraction not adhered to the anion exchange chromatography column is obtained in a loading volume (LV) of 1.5 to 2.0.

## Patentansprüche

1. Verfahren zum Entfernen des Faktors XI (FXI) während einer Plasmaproteintrennung und -reinigung, umfassend:
(a) Erhalten einer Plasmaproteinlösung, die FXI und ein Plasmaprotein enthält;
(b) Dialysieren und/oder Konzentrieren der erhaltenen Lösung;
(c) Behandeln der konzentrierten Lösung mit einem Lösungsmittel und einem Detergens, gefolgt von Durchführen einer Kationenaustauschchromatographie, um das FXI und das Plasmaprotein an eine Säule zu binden; und
(d) selektives Eluieren nur des Plasmaproteins,
wobei die Kationenaustauschchromatographie von Schritt (c) bei einem pH-Wert von 4,5 bis 5,5 und einer Flussrate von 60 cm/h durchgeführt wird, und
wobei die Schritte (c) und (d) bei einer Temperatur von 18 bis 25 °C durchgeführt werden.

2. Verfahren nach Anspruch 1, ferner umfassend Durchführen zwischen Schritt (b) und Schritt (c) einer Anionenaustauschchromatographie, um eine Fraktion zu erhalten, die nicht an einer Anionenaustauschchromatographiesäule haftet.

3. Verfahren nach Anspruch 1, wobei die Lösung von Schritt (a) durch Auflösen einer Plasmaproteinfraktion-II-Paste, gefolgt von Filtration, erhalten wird.

4. Verfahren nach Anspruch 3, wobei das Auflösen der Fraktion-II-Paste durch Zugeben einer Natriumchloridlösung in einem Volumen, das gleich dem 2- bis 10-fachen des Plasmaproteinfraktionsvolumens ist, durchgeführt wird.

5. Verfahren nach Anspruch 3, wobei die Filtration durch Klärfiltration bei einem auf 4,5 bis 5,5 eingestellten pH-Wert durchgeführt wird.

6. Verfahren nach Anspruch 1, wobei die Dialyse und/oder Konzentrierung von Schritt (b) unter Verwendung eines Ultrafiltrations-/Diafiltrations(UF/DF)-Systems durchgeführt wird und bei einem osmotischen Druck von 10 mosmol/kg oder weniger durchgeführt wird und dann ein pH-Wert auf 5,5 bis 6,5 eingestellt wird.

7. Verfahren nach Anspruch 1, wobei das Lösungsmittel von Schritt (c) Tri(n-butyl)-phosphat (TNBP) ist und ein Detergens eines oder mehrere, ausgewählt aus Polysorbat 80, Triton X-100 und Triton X-45, umfasst.

8. Verfahren nach Anspruch 1, wobei die Kationenaustauschchromatographie von Schritt (c) bei einer Salzkonzentration von 400 bis 600 mM durchgeführt wird.

9. Verfahren nach Anspruch 1, wobei eine Proteinmenge, die an der Säule der Kationenaustauschchromatographie von Schritt (c) adsorbiert ist, 90 bis 130 mg/ml pro ml eines Kationenaustauschharzes beträgt.

10. Verfahren nach Anspruch 1, wobei die Kationenaustauschchromatographie von Schritt (c) unter Verwendung eines Kationenaustauschharzes auf Keramikbasis durchgeführt wird.

11. Verfahren nach Anspruch 1, wobei in Schritt (d) nur das Plasmaprotein unter Verwendung von 400 bis 600 mM Natriumchlorid (NaCl) selektiv eluiert wird.

12. Verfahren nach Anspruch 1, ferner umfassend Durchführen einer Reinigung vor Ort (CIP), um nach Abschluss der Elution des Plasmaproteins in Schritt (d) das Kationenaustauschharz unter Verwendung von Natriumhydroxid (NaOH) zu waschen.

13. Verfahren nach Anspruch 12, wobei die Reinigung vor Ort (CIP) bei einer Natriumchloridkonzentration von 200 bis 1.000 mM durchgeführt wird.

14. Verfahren nach Anspruch 2, wobei die Anionenaustauschchromatographie bei einem pH-Wert von 5,5 bis 6,5 und einer Flussrate von 95 bis 145 cm/h durchgeführt wird und eine Fraktion, die nicht an der Anionenaustauschchromatographiesäule haftet, in einem Beladungsvolumen (LV) von 1,5 bis 2,0 erhalten wird.

## Revendications

1. Procédé d'élimination du facteur XI (FXI) lors de la séparation et de la purification de protéines plasmatiques, comprenant :
(a) l'obtention d'une solution de protéines plasmatiques contenant du FXI et une protéine plasmatique ;
(b) la dialyse et/ou la concentration de la solution obtenue ;
(c) le traitement de la solution concentrée avec un solvant et un détergent, puis la réalisation d'une chromatographie à échange de cations pour lier le FXI et la protéine plasmatique à une colonne ; et
(d) l'élution sélective de la protéine plasmatique uniquement,
dans lequel la chromatographie à échange de cations de l'étape (c)
est effectuée à un pH de 4,5 à 5,5 et à un débit de 60 cm/h, et dans lequel les étapes (c) et (d) sont réalisées à une température de 18 à 25 °C.

2. Procédé selon la revendication 1, comprenant en outre la mise en oeuvre d'une chromatographie à échange d'anions pour obtenir une fraction n'adhérant pas à une colonne de chromatographie à échange d'anions entre l'étape (b) et l'étape (c).

3. Procédé selon la revendication 1, dans lequel la solution de l'étape (a) est obtenue par dissolution d'une pâte de fraction II de protéine plasmatique, suivie d'une filtration.

4. Procédé selon la revendication 3, dans lequel la dissolution de la pâte de fraction II est réalisée par ajout d'une solution de chlorure de sodium dans un volume égal à 2 à 10 fois le volume de fraction de protéine plasmatique.

5. Procédé selon la revendication 3, dans lequel la filtration est réalisée par filtration clarifiante à un pH ajusté pour atteindre 4,5 à 5,5.

6. Procédé selon la revendication 1, dans lequel la dialyse et/ou la concentration de l'étape (b) est réalisée en utilisant un système d'ultrafiltration/diafiltration (UF/DF) et est menée à une pression osmotique inférieure ou égale à 10 mOsmol/kg et le pH est ensuite ajusté pour atteindre 5,5 à 6,5.

7. Procédé selon la revendication 1, dans lequel le solvant de l'étape (c) est le tri(n-butyl)-phosphate (TNBP) et un détergent comprend un ou plusieurs sélectionnés parmi le polysorbate 80, le Triton X-100 et le Triton X-45.

8. Procédé selon la revendication 1, dans lequel la chromatographie à échange de cations de l'étape (c) est réalisée à une concentration en sel de 400 à 600 mM.

9. Procédé selon la revendication 1, dans lequel une quantité de protéine adsorbée sur la colonne de la chromatographie à échange de cations de l'étape (c) est de 90 à 130 mg/ml par ml d'une résine échangeuse de cations.

10. Procédé selon la revendication 1, dans lequel la chromatographie à échange de cations de l'étape (c) est réalisée en utilisant une résine échangeuse de cations à base de céramique.

11. Procédé selon la revendication 1, dans lequel, à l'étape (d), seule la protéine plasmatique est éluée sélectivement en utilisant 400 à 600 mM de chlorure de sodium (NaCl).

12. Procédé selon la revendication 1, comprenant en outre la réalisation d'un nettoyage en place (CIP) pour laver la résine échangeuse de cations en utilisant de l'hydroxyde de sodium (NaOH) après l'achèvement de l'élution de la protéine plasmatique à l'étape (d).

13. Procédé selon la revendication 12, dans lequel le nettoyage en place (CIP) est effectué à une concentration en chlorure de sodium de 200 à 1 000 mM.

14. Procédé selon la revendication 2, dans lequel la chromatographie à échange d'anions est réalisée à un pH de 5,5 à 6,5 et à un débit de 95 à 145 cm/h et une fraction non adhérée à la colonne de chromatographie à échange d'anions est obtenue dans un volume de chargement (LV) de 1,5 à 2,0.
